(19) Europäisches Patentamt / European Patent Office / Office européen des brevets

(11) **EP 3 332 693 A1**

(12) **EUROPEAN PATENT APPLICATION**

(43) Date of publication:
**13.06.2018  Bulletin 2018/24**

(51) Int Cl.:
**A61B 5/00** (2006.01)　　　**G01N 29/07** (2006.01)
**A61F 2/30** (2006.01)

(21) Application number: **16202409.5**

(22) Date of filing: **06.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **ETH Zurich**
**8092 Zurich (CH)**

(72) Inventors:
• **Taylor, William**
**8955 Oetwil an der Limmat (CH)**
• **Vogl, Florian**
**8047 Zürich (CH)**

(54) **SYSTEM AND METHOD FOR NON-INVASIVE EVALUATION OF PROSTHESIS LOOSENING, AND/OR FRACTURE HEALING**

(57)　Disclosed embodiments concern a system for monitoring one or more mechanical properties of an assembly of rigid bodies, comprising: at least one first transducer that is operative to excite mechanical waves that propagate from a first rigid body of the assembly via a contact interface to a second rigid body of the assembly; at least one distant sensor that is operative to sense one or more physical stimuli relating to the mechanical waves propagating in the second rigid body; a memory; and a processor that is operative to execute instructions stored in the memory to perform the following steps: receiving electronic signals that relate to the mechanical waves propagating in the first and second rigid body; and determining, based on the received electronic signals, a state and/or a change in a state of a structural feature of the assembly.

FIG. 1

EP 3 332 693 A1

**Description**

**TECHNICAL FIELD AND BACKGROUND**

Embodiments disclosed herein relate in general to systems, apparatuses and methods for the non-invasive evaluation of prosthesis loosening and/or fracture healing.

Various types of prostheses that have been proven useful in improving the quality of life of a patient are known in the art. Such prostheses may for example include artificial joint and/or bone implants (also: replacements) which are subjected to everyday motion, stress and strain, which may lead to abrasion, loosening, and/or wear between different parts of the implant and/or between the implant and portions of the skeletal frame. As a result thereof, the quality of the interface between the artificial joint and/or bone implant(s) may begin to deteriorate, ending in possible implant failure (also: malfunction).

One of the most common forms of artificial joint and/or bone implant malfunction processes includes "aseptic loosening". Aseptic loosening is, as the name implies, a loosening of the implant without the occurrence of an infection. This type of implant malfunction is one of the most common reasons for revision of e.g. total knee replacements/arthroplasties, with aseptic loosening causing around 39.9% of revisions scheduled.

Current methods to detect aseptic loosening involve X-ray or nuclear imaging such as, for instance, Single-Photon Emission Computed Tomography (SPECT). These methods expose the patient to ionizing radiation, require expensive equipment and specially trained personnel, and are invasive when the employment of a contrast agent is required such as in SPECT, or used to improve accuracy. These methods are therefore unsuitable for widespread preventative or monitoring applications. In addition, while the imaging of metal of an implant with X-ray may introduce artefacts, metal may not be imaged with MRI. Furthermore, the above-noted imaging methods may be adversely affected due to field of view (FOV) obstruction by metal. The above-noted difficulties thus impede the detection of implant loosening and possibly even the detection of total implant failure.

Moreover, these imaging methods are quite slow and may in some instances require up to 1 hour of medical examination. Currently, with the aforementioned imaging methods, only a 90% true-positive rate may be obtainable, even under optimal examination conditions. Hence, at least 1 out of 10 surgical revisions is eventually performed unnecessarily, with all the associated medical, social, and economic implications.

**GENERAL DESCRIPTION**

The following description of the apparatus, system and method is given with reference to particular examples, with the understanding that such system, apparatus and/or method is not limited to these examples.

The disclosed embodiments relate to a system, apparatus and method for non-invasively monitoring mechanical properties of an assembly of two or more rigid bodies.

The expression "rigid" as used herein also encompasses the term "substantially rigid". It should further be noted that the expressions "assembly of two or more rigid bodies" and "rigid body assembly" should not be construed as to exclude flexible bodies. In other words, an assembly of two or more rigid bodies may, in some embodiments, also comprise flexible structures and/or elements such as, for example, tendons and ligaments. In some embodiments, the first and/or the second rigid body can be a prosthesis or a bone.

An assembly of two or more rigid bodies may be formed, for example, by articulating one rigid body with another rigid body to form an articulated joint allowing movement of the bodies relative to each other, or by the fusion of two or more rigid bodies with each other to obtain a fixed or fused connection so that the bodies are rigidly coupled or structurally integrated with each other. A fixed or fused connection between two or more rigid bodies may for example be obtained through osseointegration. In other words, an assembly may in one embodiment refer to or comprise a "static joint", i.e., a joint in which the rigid bodies have no degrees of freedom between them; and in another embodiment refer to or comprise an articulated joint, in which there is at least one degree of freedom (e.g., rotation around at least one axis expressible by an Euler angle).

An assembly of two or more rigid bodies (including for instance an articulated joint and/or a fracture healing site) may be monitored, for example, to non-invasively and automatically detect prosthesis loosening and/or delayed fracture healing and/or non-union. It is noted that the "monitoring of a mechanical property or properties" may include or encompass the meaning of the expression "monitoring of a structural feature of the assembly", and includes determining a value relating to a parameter of the structural feature for determining a state and/or a change in the state thereof. Such structural feature may include a contact interface between two rigid bodies of the assembly, for example, to evaluate the degree to which an implant is anchored or not anchored in a bone and/or bone fracture site. Optionally, the system, apparatus and method disclosed herein may be employed to monitor the mechanical properties of a rigid body, e.g., to detect crack formation in an implant.

It should be noted that in the discussion herein, the term "prosthesis" may include an artificial replacement of a body part, either internal or external. In case the prosthesis is an artificial replacement of an internal body part (e.g., of a mammalian subject), it may be referred to as an "implant". It should be noted that the term "body part" may also refer to a body of a robot for example, and/or any other structure (movable or non-movable) for which the non-destructive testing of assemblies may be of interest. Therefore, while certain embodiments may be discussed in conjunction with an implant-bone contact interface, this should by no means be construed limiting, and embodiments disclosed herein may also be employed, for example, in the context of one or more contact interfaces formed between surface portions of two or more implants, two or more bones and between two sections of a fractured bone. Moreover, embodiments disclosed herein may also relate to external artificial replacements (also: external prosthesis) forming an interface with a patient's residual limb portion.

In an embodiment, the system for determining a state and/or change in state of a contact interface between a first and a second rigid body comprises at least one first mechanical wave generator (also: transducer) that is operative to excite mechanical waves that, initially, propagate from the first rigid body via the contact interface to the second rigid body. The terms "mechanical wave" and "acoustic wave" may herein be used interchangeably. The system further comprises at least one distant sensor that is operative to convert a physical stimulus relating to mechanical waves (e.g., including initial and/or reflected waves) that propagated from the first rigid body via the contact interface to the second rigid body into an electronic signal; a memory; and a processor that is operative to execute instructions stored in the memory to perform the following steps: determining, based on the electronic signal produced by the at least one distant sensor, one or more values that relate to one or more parameters of mechanical waves propagating in the second rigid body; and determining, based on the value(s), a state and/or a change in a state of the contact interface.

In an embodiment, a near sensor may be employed that is, with respect to the contact interface of the assembly, arranged on the same side as or ipsilateral with respect to the first transducer.

The at least one near and/or distant sensor may comprise any one of the following: an accelerometer; a velocity sensor; a strain gauge; acoustic sensors and/or a displacement sensor.

In an embodiment, the system comprises at least one second transducer that is operative to excite mechanical waves that, initially, propagate from the second rigid body via the contact interface to the first rigid body. With respect to the contact interface, the at least one second transducer is located contralateral both to the at least one first transducer and the at least one near sensor. Correspondingly, the at least one second transducer operably engages the same side of the assembly as the at least one distant sensor.

In an embodiment, the at least one first and/or second transducers and/or the at least one near and/or distant sensor are removably or non-removably attachable to: a skin portion of a mammalian subject; a bone of a mammalian subject; an implant; bone pins; and/or any other tissue of the mammalian subject.

The term "reference sensor" as used herein refers to a sensor that is positioned on the ipsilateral side of a contact interface formed between two rigid bodies of an assembly as a given transducer. In other words, the reference sensor and the given transducer are located on the same side of the same rigid body that forms the assembly with another rigid body. Due to the ipsilateral positions and operative engagements of the reference sensor and the given transducer, the reference sensor can be operative to sense physical quantities of mechanical waves propagating in the same rigid body away from the given transducer towards the reference sensor before crossing the contact interface. Moreover, the reference sensor can be operative to sense physical quantities of mechanical return waves excited by the given transducer and reflected from the contact interface and/or the other rigid body towards the same rigid body where the reference sensor is located. In view of the aforesaid, the near sensor may herein also be referred to as a "first reference sensor" with respect to mechanical waves excited by the first transducer, and the distant sensor may be referred to as a "second reference sensor" with respect to mechanical waves excited by the second transducer. In an embodiment, such (first and/or second) reference sensor, which senses a physical quantity of the mechanical waves before propagating through the interface for the first time, may provide a feedback signal to a transducer ipsilateral to the reference sensor to adjust for inter-patient differences in bone/implant structure, properties, geometry, and/or soft tissue structure and/or the like. More specifically, based on the feedback signal, the transducer that is ipsilateral to the reference sensor provides or produces, responsive to the received feedback, an excitation input into assembly 200 to excite in the rigid body ipsilateral to the exciting transducer, the propagation of a "standardized" mechanical wave. In other words, the transducer's excitation input can be selected so that, in response, the physical properties of mechanical waves excited in the rigid bodies that are ipsilateral to the exciting transducers, as sensed by the reference sensors ipsilateral to the transducers, have about the same physical properties (within a certain margin of error), despite possible inter-patient differences in the mechanical properties of the rigid assemblies. Accordingly, when the first transducer and first reference sensor is used, the wave in the first rigid body is standardized, while if the second transducer and second reference sensor is used, the wave in the second body is standardized.

As already indicated herein, a state and/or a change in a state of a structural feature of the assembly may be determined based on the value(s) relating to a physical quantity of mechanical waves excited by the first transducer and sensed by the distant sensor.

Optionally, a state and/or a change in state of the of a structural feature of the assembly may be determined based on values relating to a physical quantity of mechanical waves sensed by the near sensor, which is located ipsilaterally with respect to the first transducer. In an embodiment, the physical quantity of mechanical waves sensed by the near sensor may relate to mechanical waves excited by the first transducer and, optionally, by the second transducer positioned ipsilaterally with respect to the distant transducer.

The expressions "sensing a physical stimulus" and "sensing a physical quantity" as used herein, as well as grammatical variations thereof, may herein be used interchangeably.

As already indicated, the terms "mechanical wave generator" and "transducer" may herein be used interchangeably. The term "transducer" as used herein refers to a device that is operative to convert one form of energy (e.g., electrical signals) into mechanical waves.

In an embodiment, the at least one first transducer and the at least one distant sensor can be positioned to measure a flux of acoustic energy from the first to the second rigid body via the contact interface in response to exciting the propagation of mechanical waves in the first rigid body. In an embodiment, the system is operative to measure a change in flux of acoustic energy.

The distant sensor for example is positioned and operative to sense mechanical waves that have propagated via or through the contact interface. For example, the at least one first transducer and the at least one distant sensor may be positioned on opposite sides of the contact interface such that the at least one distant sensor can sense acoustic or mechanical energy that propagated through the contact interface.

In view of the aforesaid, the term "distant" as used herein refers to a position of a sensor relative to the first rigid body to indicate that such distant sensor is positioned to sense physical quantities of mechanical waves that propagate away from the first transducer positioned at the first rigid body, via the contact interface, towards the second rigid body where the distant sensor is located.

It should be noted that the term "physical stimuli" may be considered to be equivalent to term "physical quantity". Furthermore, the term "propagating in" may also encompass the meaning of the terms "propagating through" and "propagating via", as well as corresponding grammatical variations thereof.

A "state of a contact interface" may for example relate to a level of correspondence, level of congruency, a quality of an interface, stability, strength, stiffness, rigidity and/or a level of surface conformity between surface portions of the at least two rigid bodies.

In an embodiment, with respect to an articulated joint, a state of a structural feature of the assembly may relate to static and/or dynamic frictional coefficients of the contacting surfaces of the articulated rigid bodies. For example, an increase and/or decrease, and/or an unevenness in (static and/or dynamic) frictional coefficients that is dependent on the orientation between two articulated bodies may be an indication of a decrease in level of congruency between the surface portions of the two articulated rigid bodies. With respect to bodies of an assembly that are supposed to become rigidly coupled with each other such as in fracture healing, increased movement between the bodies or looseness of the assembly may be indicative of a reduction in the quality of the contact interface and can relate, for example, to the ability of the at least two rigid bodies to move relative to each other. In other words, increased stiffness, stability and/or lack of ability to move the at least two rigid bodies relative to each other are indicative of increased quality of the contact interface.

Accordingly, a value or values relating to a state of a contact interface may be indicative of the looseness and/or robustness of an assembly formed by, *inter alia,* rigid bodies, and/or the level of functional and/or operational stability of the assembly, e.g., depending on the desired functional purpose of the assembly.

In an embodiment, and as will be outlined herein below in more detail, the value or values may relate to one or more parameters of mechanical waves that propagate from the first rigid body over the contact interface to the second rigid body, and/or vice versa. Such value or values may, for example, be descriptive of energy, phase, frequency, amplitude, power spectrum, change in the amplitude (e.g., attenuation), change in the power spectrum, amplitude ratio, change in an amplitude ratio, and/or excitation energy vs energy of higher harmonics of the propagating mechanical waves under various assembly loading conditions and/or the like.

It is noted that the surfaces of the rigid bodies forming the contact interface may be in direct or indirect contact with each other. The expression "indirect contact" as used herein may refer to cases in which, for example, there is a layer interposed between two rigid bodies of the assembly, e.g., for cushioning or damping purposes. The expression "direct contact" may refer to cases in which the surfaces of rigid bodies are in physical contact with each other.

In an embodiment, the memory is operative to receive data that is descriptive of the assembly's loading condition. Such data may for example be provided manually by a medical professional via a user interface. In another embodiment, the processor, in conjunction with the memory, may receive sensor data from sensors (including, e.g., inertial sensors) that are coupled to the patient and determine, based on the received sensor data (which can include inertial sensor data), the loading condition. The processor may thus automatically detect an occurrence of a loaded and unloaded loading condition and classify the received electronic signals accordingly for further processing.

Based on the flux of acoustic energy measured across the contact interface under two or more different loading conditions, and further based on the information about the loading conditions, the processor is operative to determine the state and/or change in state of a structural feature of the assembly such as, for example, the contact interface.

The different loading conditions, which are herein classified as "relaxed condition" and "loaded condition", may be defined with respect to a difference in the load to which the contact interface can be expected to be correspondingly subjected to. For the purpose of determining a state and/or a state in the contact interface, the relaxed and loaded conditions have to be sufficiently different from each other with respect to the loading imparted on the assembly (and, therefore, on the contact interface). Expressed in percentages, differences between a loaded condition and a relaxed condition that allow determining a state and/or a change of state of the contact interface may range, for example, from 100 % bodyweight (e.g., standing) to 20% of bodyweight (e.g., sitting); from 300% bodyweight (e.g., one-legged stance) to 20% of bodyweight (e.g., sitting), in the loaded condition from 30%. 40%, 50%, 60%, 70%, 80%, 200%, 400 %, 500%, 600%, 700%, 800%, 900%, 1000% of body weight to 20%, 15% or 10% of the bodyweight in the relaxed condition. The bodyweight which may in some embodiments be the weight of the assembly. Expressed in nominal weight values, the minimum required differences in the loads imparted in the loaded and relaxed conditions for determining a state and/or a change in the state of the contact interface may be, for example, at least 5 kg, 10 kg, 15 kg, 20 kg, 30 kg, 40 kg, 50 kg, 60 kg, 70 kg, 80 kg, 90 kg, 100 kg, 110 kg, 120 kg, 130 kg, 140 kg, 150 kg, 160 kg, 170 kg, 180 kg, 190 kg, 200 kg, 210 kg, 220 kg, 230 kg, 240 kg, 250 kg, 260 kg, 270 kg, 280 kg, 290 kg, or, at least, 300 kg. The maximum applicable load is defined by a (damaging) load that would, if applied, cause damage to the structure of the assembly. The maximum applicable load is thus slightly below that damaging load, e.g., by 500 grams, 1 kg or 2 kg. In percentage, the maximum applicable load may be, for example, 95% or less of the damaging load. While herein an object's weight is expressed in "Kilograms", it actually refers to "kilogram-force, with the force being about 9.8 m/s$^2$ for the purposes of the applications disclosed herein.

A relaxed condition can be obtained when an unloading, relaxation or unstress activity is performed on the assembly that is expected to reduce load on the assembly compared to the load in the loaded condition. Conversely, the loaded condition can be obtained when a loading activity is performed that is expected to increase load on the assembly compared to the load in the relaxed condition. An assembly can be considered to be in "a relaxed condition", for example, when the patient is sitting or supine, as opposed to be in a "loaded condition", for example, when the patient is standing. Exemplarily, these activities may be performed by the patient itself by altering the position, and/or by a medical professional subjecting the assembly to different loads.

Based on the flux of acoustic energy measured when the assembly is under each one of the two or more loading conditions of the same patient, the mechanical properties of the patient's assembly may be determined.

In some embodiments, the system, apparatus and method disclosed herein may be employed to monitor over a prolonged period of time the mechanical properties of an assembly, e.g., to monitor a fracture healing site. Optionally, the system may be configured to allow the monitoring of mechanical properties of an assembly at multiple arbitrary follow-up time points, e.g., at least 12 hours, 24 hours, 2 days, 3 days, 4 days, 5 days, 6 days, 1 week, 2 weeks, 3 weeks, 4 weeks, 6 weeks, 2 months, 3 months, or for at least 4 four months.

Additional example implementations

Example 1 concerns a system for monitoring one or more mechanical properties of an assembly of rigid bodies, comprising: at least one first transducer that is operative to excite mechanical waves that propagate from a first rigid body of the assembly via a contact interface to a second rigid body of the assembly; at least one distant sensor that is operative to sense one or more physical stimuli relating to the mechanical waves propagating in the second rigid body; a memory; and a processor that is operative to execute instructions stored in the memory to perform the following steps: receiving, while the assembly is under a loaded condition and also while the assembly is under a relaxed condition, electronic signals that relate to the mechanical waves propagating in the first and second rigid body; and determining, based on the received electronic signals, a state and/or a change in a state of a structural feature of the assembly, wherein the electronic signals comprise: a) an electronic signal provided by the distant sensor; and at least one of the following: b) an electronic signal provided to the at least one first transducer to excite the propagation of mechanical waves; and c) an electronic signal provided by a near sensor located contralaterally to the distant sensor. The electronic signals can relate to one or more values of one or more parameters of the mechanical waves propagating in the assembly.

Example 2 includes the subject matter of example 1 and, optionally, wherein the structural feature is the contact interface.

Example 3 includes the subject matter of any one of examples 1 to 2 and, optionally, wherein the at least one first transducer and the at least one distant sensor are positioned on opposite sides of the contact interface.

Example 4 includes the subject matter of any one of examples 1 to 3 and, optionally, wherein the at least one first transducer and the at least one distant sensor can be positioned to measure a flux of acoustic energy from the first to the second rigid body via the contact interface, wherein the energy flux is caused responsive to exciting, by the at least one first transducer, the propagation of mechanical waves in the first rigid body. In other words, the energy flux is generated by the first transducer exciting the propagation mechanical waves in the first rigid body.

Example 5 includes the subject matter of any one of examples 1 to 4 and, optionally, wherein the system is operative to associate classifications with the received electronic signals as occurrences of one of the following: a loaded loading condition, and a relaxed loading condition to which the assembly is subjected to; wherein the classification of the received electronic signals is performed automatically, by the processor executing instructions stored in the memory, based on signals received from sensors (e.g., inertial sensors) that are coupled to a patient comprising the assembly and by comparing data relating to the signals received from the sensors with loading condition data stored in the memory; or based on an input provided by a medical professional via a user interface of the system. The received electronic signals may then be further analyzed according to their classification for determining the state and/or change of state of a mechanical property of the assembly. For example, electronic signals that are classified as "loaded condition" may be compared against electronic signals classified as "relaxed condition", e.g., by calculating a ratio between the respective values.

Example 6 includes the subject matter of any one of the examples 1 to 5 and, optionally, wherein the received electronic signals relate to energy, phase, and/or frequency of the propagating mechanical waves.

Example 7 includes the subject matter of any one of the examples 1 to 6 and, optionally, at least one second transducer that is operative to excite mechanical waves that propagate from the second rigid body via the contact interface to the first rigid body.

Example 8 includes the subject matter of any one of the examples 1 to 7 and, optionally, wherein the at least one first and/or second transducers areoperative to excite, in the assembly, the propagation of mechanical waves having a plurality of amplitudes, phases, and/or excitation frequencies.

Example 9 includes the subject matter of any one of the examples 1 to 8 and, optionally, wherein the determined state and/or change of state of the contact interface includes a level of physical conformity between a surface of the first rigid body and a surface of the second rigid body forming the contact interface.

Example 10 includes the subject matter of any one of the examples 1 to 9 and, optionally, wherein based on a non-conformity criterion, the processor provides an output indicative of a state and/or change of state of the contact interface.

Example 11 includes the subject matter of any one of the examples 1 to 10 and, optionally, wherein the at least one near and/or at least one distant sensor is employed to provide a reference value to reduce noise in the electronic signal of the at least one near and/or distant sensor and/or to control the at least one first and/or second transducer such to excite, in the respective rigid body, the propagation of a standardized mechanical wave.

Example 12 concerns a method for monitoring one or more mechanical properties of an assembly of rigid bodies, comprising:

exciting, while the assembly is in a loaded condition, the propagation of mechanical waves in the assembly from a first rigid body to a second rigid body of the assembly via a contact interface therebetween;
sensing physical quantities relating to the mechanical waves propagating in the assembly when the assembly is in the loaded condition;
producing first electronic signals relating to the sensed physical quantities of the mechanical waves propagating in the assembly when the assembly is the loaded condition;
exciting, while the assembly is in a relaxed condition, the propagation of mechanical waves in the assembly from the first rigid body to the second rigid body of the assembly via the contact interface therebetween;
sensing physical quantities relating to the mechanical waves propagating in the assembly when the assembly is in the relaxed condition;
producing second electronic signals relating to the sensed physical quantities of the mechanical waves propagating in the assembly when the assembly is in the relaxed condition;
determining, based on the first and second electronic signals, a state and/or a change in a state of a mechanical property of the assembly,
wherein the first and second electronic signals each comprise: a) an electronic signal provided by the distant sensor; and at least one of the following: b) an electronic signal provided to the transducer to excite the propagation of mechanical waves; and c) an electronic signal provided by a near sensor located contralaterally to the distant sensor.

Example 13 concerns a computer program product comprising a program code for the execution of the method steps according to example 12, wherein the computer program product is executed on a computer.

Example 14 concerns the use of the system according to any one of the examples 1 to 11.

## DESCRIPTION OF THE FIGURES

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. The number of elements shown in the Figures should by no means be construed as limiting and is for illustrative purposes only. The figures are listed below.

**FIG. 1** is a schematic illustration of a system that is in operative association with at least two rigid bodies of an assembly comprised in a patient and employed for monitoring a structural feature of an assembly, according to an embodiment;

**FIG. 2** is a schematic block diagram illustration of the system, according to an embodiment;

**FIG. 3A** is a schematic illustration of the patient in a body position in which the assembly is in a loaded condition;

**FIG. 3B** is a schematic illustration of the patient in another body position in which the assembly is relaxed compared to the body position shown in **FIG. 1B;**

**FIG. 4** is a flow chart illustration of a method for monitoring a structural feature of the assembly;

**FIG. 5A** shows a photograph of an experimental setup to assess the efficacy of the method in a test assembly;

**FIG. 5B** shows a more detailed photograph of the test assembly;

**FIG. 6** shows for a first transducer position a diagram that illustrates a measurement of the ratio of the average energy of the first harmonic to the base excitation frequency as a function of fixation state and mechanical loading condition (under a loaded and relaxed (loading) condition); and

**FIG. 7** shows, for a second transducer position, a diagram that illustrates, in relaxed condition, the average energy of the first harmonic to the base frequency as a function of fixation state.

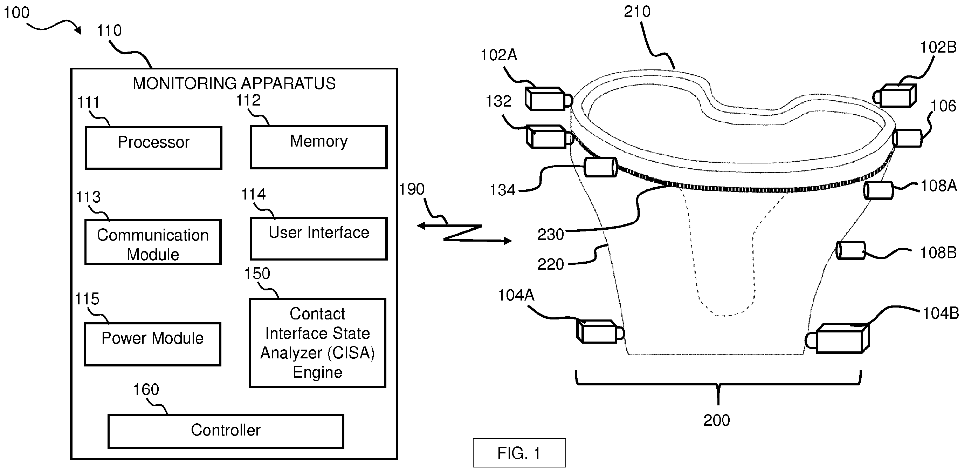

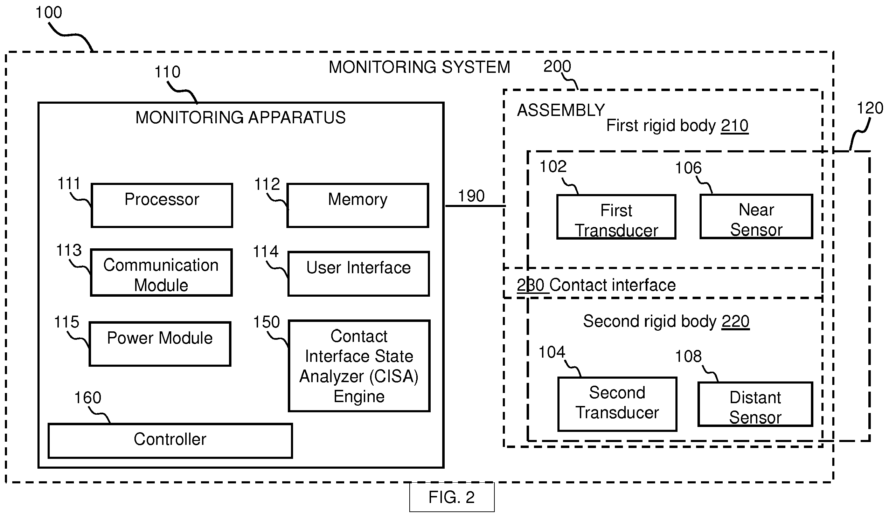

Reference is made to **FIGs. 1** and **2.** Generally, system **100** may be operative to enable the implementation of a method, process and/or operation for monitoring a mechanical property of an assembly of rigid bodies including, for example, the monitoring of a structural feature of the assembly comprising, e.g., one or more contact interfaces formed between two or more rigid bodies. In **FIG. 2A,** elements that are not part of system **100** are outlined with broken lines.

The two or more rigid bodies may be articulated with each other by a joint. Alternatively, the two or more rigid bodies are or are supposed to become rigidly coupled with each other. System **100** is for example operative to determine a state and/or a change in state of a contact interface formed by a surface of the first rigid body and a surface of the second rigid body. The monitoring of a contact interface comprises determining a state and/or change of a state of the contact interface.

System **100** is further configured to enable the implementation of a method, process and/or operation for monitoring, e.g., osseointegration and further to predict and/or detect incorrect structural and functional connection between, for example, a bone and an artificial implant.

While some of the embodiments are herein described in conjunction with an articulated joint, this should by no means be construed limiting. Accordingly, system **100** and the associated method described herein may be also be employed in conjunction with non-articulated joints in assemblies that are formed through fusion (e.g., osseointegration) of rigid bodies.

As schematically and exemplarily shown in **FIG. 1,** system **100** may be operably coupleable with an assembly **200** (e.g., a tibial tray and bone assembly) for forming the basis of, for example, an articulating joint. Assembly **200** comprises first and second rigid bodies **210** and **220** such that a state and/or a change of state of a contact interface **230** between the rigid bodies is measurable by system **100,** as outlined herein below in greater detail. Exemplarily in correspondence with the illustration shown in **FIG. 1,** first rigid body may herein also be referred to as an "implant", and second rigid body **220** may herein also be referred to as "bone".

It should be noted that while the discussion that follows refers to embodiments that address a single interface formed between two rigid bodies, this should by no means be construed as limiting. For example, embodiments disclosed herein may enable the monitoring of a contact interface of various types of assemblies comprising, for example, non-articulated joints and/or articulated joints. Non-limiting examples of non-articulated (also: fixed or fused connections) rigid body connections include fracture healing and osseointegrations sites. Accordingly, system **100** allows the monitoring of fracture healing sites over a prolonged period of time.

Non-limiting examples of articulated joints include fibrous joints, cartilaginous joints and/or synovial joints. Types of synovial joints that may be monitored by system **100** can include, for example, a hinge-type, a pivot-type, a ball-and-socket type, a saddle-type, a condyloid, and/or a gliding joint. Further, system **100** disclosed herein may enable the monitoring of a contact interface formed by a simple joint, a compound joint and/or of a complex joint.

System **100** comprises at least one first transducer **102** that is operative to excite mechanical waves that propagate from first rigid body **210** via contact interface **230** to second rigid body **220**. Some of the mechanical waves excited by the at least one first transducer may be reflected by contact interface **230** and/or some may be reflected from second rigid body **220**. In an embodiment, system **100** may further comprise at least one second transducer **104** to excite mechanical waves that propagate from second rigid body **220** via contact interface **230** to first rigid body **210b,** some of the mechanical waves excited by the at least one second transducer may be reflected by contact interface **230** and/or some may be reflected from first rigid body **210.**

System **100** includes at least one distant sensor **108** that is operative to convert a physical stimulus relating to mechanical waves propagating in second rigid body **220** into an electronic signal. In an embodiment, system **100** further includes at least one near sensor **106** that is operative to convert a physical stimuli relating to mechanical waves propagating in first rigid body **210** into a corresponding electronic signal.

System **100** may include an apparatus **110** that may include a processor **111,** a memory **112,** a communication module **113,** a user interface **114,** a contact interface state analyzer (CISA) engine **150,** and a power module **115** for powering the various components and/or engines of system **100.** User interface **114** may for example allow a medical professional to provide system **100** with information about a loading condition, and allow the medical professional to receive information about a state and/or a change in a state of a structural feature of the assembly.

Optionally, monitoring apparatus **110** may comprise a controller **160** for controlling the operation of various components of system **100** such as, for example, the transducers.

The various components of system **100** may communicate with each other over one or more communication buses (not shown) and/or wired and/or wireless signal links. For example, components of monitoring apparatus **110** may communicate with first and second transducers **102** and **104** as well as with near and distant sensors **106** and **108** over a communication network **190.**

The term "processor", as used herein, may additionally or alternatively refer to a controller. Processor **111** may be implemented by various types of processor devices and/or processor architectures including, for example, embedded processors, communication processors, graphics processing unit (GPU)-accelerated computing, soft-core processors and/or general purpose processors. Moreover, a controller may be implemented by a processor.

Memory **112** may include one or more types of computer-readable storage media such as, for example, transactional memory and/or long-term storage memory facilities and may function as file storage, document storage, program storage, and/or as a working memory. The latter may for example be in the form of a static random access memory (SRAM), dynamic random access memory (DRAM), read-only memory (ROM), cache or flash memory. As long-term memory they may for example include a volatile or non-volatile computer storage medium, a hard disk drive, a solid state drive, a magnetic storage medium, a flash memory and/or other storage facility. A hardware memory facility may for example store a fixed information set (e.g., software code) including, but not limited to, a file, program, application, source code, object code, and the like. As working memory, memory **112** may, for example, process instructions including, e.g., temporally-based and/or non-temporally based instructions.

Communication module **113** may, for example, include I/O device drivers (not shown) and network interface drivers (not shown) for enabling the transmission and/or reception of data over a network **190.** A device driver may for example, interface with a keypad or to a USB port. A network interface driver may for example execute protocols for the Internet, or an Intranet, Wide Area Network (WAN), Local Area Network (LAN) employing, e.g., Wireless Local Area Network (WLAN), Metropolitan Area Network (MAN), Personal Area Network (PAN), extranet, 2G, 3G, 3.5G, 4G including for example Mobile WIMAX or Long Term Evolution (LTE) advanced, Bluetooth® (e.g., Bluetooth smart), ZigBee™, near-field communication (NFC) and/or any other current or future communication network, standard, and/or system.

User Interface **114** may, for example, comprise a keyboard, a touchscreen, and/or the like.

Power module **115** may, for example, comprise an internal power supply (e.g., a rechargeable battery) and/or an interface for allowing connection to an external power supply.

CISA engine **150** may be realized by one or more hardware, software and/or hybrid hardware/software modules, e.g., as outlined herein. A module may be a self-contained hardware and/or software component that interfaces with a larger system and may comprise a machine or machines executable instructions. For example, a module may be implemented as a controller programmed to, or a hardware circuit comprising, e.g., custom VLSI circuits or gate arrays, off-the-shelf semiconductors such as logic chips, transistors, or other discrete components, operative to cause system **100** to implement the method, process and/or operation as disclosed herein. A module may also be implemented in programmable hardware devices such as field programmable gate arrays, programmable array logic, programmable logic devices or the like. For example, memory **112,** may include instruction which, when executed e.g. by the processor **111,** may cause the execution of a method, process and/or operation for determining a state and/or a change of state of contact interface **230.**

In an embodiment, CISA engine **150** may be operative to determine, based on the measured values relating to parameters of mechanical waves propagating via contact interface **230** that are received by distant sensor **108** and, optionally, by near sensor **106,** a state and/or change of state of a contact interface formed by a surface of first rigid body **210** and a surface of second rigid body **220.** For example, CISA engine **150** may provide an output indicative of a reduction in conformity between the first and second surface. For instance, CISA engine **150** may provide an output that is indicative of a looseness of assembly **200.**

In an embodiment, the at least one first transducer **102** and/or the at least one second transducer **104** may be operative to excite in assembly **200** the propagation of mechanical waves that have one or more baseband frequencies, phases, and/or amplitudes.

In an embodiment, the at least one first transducer **102** and/or the at least one second transducer **104** may be operative to provide, produce, or subject a rigid body to an excitation input having a plurality of amplitudes, phases, and/or excitation frequencies.

A main input frequency provided to transducers **102** and/or **104** may herein be referred to as "central excitation frequency" having corresponding widths, while the frequencies of mechanical waves propagating in first and second rigid bodies **210** and **220** as well as through contact interface **230** of articulated assembly **200** may herein be referred to as "baseband frequencies". In other words, a baseband frequency of a propagating mechanical wave is excited by a corresponding central excitation frequency.

In an embodiment, a central excitation frequency may range, for example, from 2 kHz to 8 kHz. In an embodiment, a central excitation frequency may be input to articulated assembly **200** in a continuous or pulsed manner. In an embodiment, a range of central excitation frequencies may be provided in a sweeping manner. In an embodiment, a range of central excitation frequencies may be provided concurrently. For example, one or more first and second transducers **102** and **104** may input a range of central excitation frequencies. In an embodiment, an input pulse may be provided having a width ranging, for example, from 50 Hz to 1 kHz.

In an embodiment, the at least one first and/or second transducers **102** and **104** may be operative to excite in the first and/or second rigid body **210** and/or **220,** responsive to controllably providing an input of one or more central excitation frequencies, the propagation of mechanical waves having corresponding baseband frequencies and possibly, depending on the assembly's mechanical properties (e.g., the state of the contact interface), one or more higher order harmonics.. For example, a central excitation frequency of 7 kHz may cause the propagation of a mechanical wave at a baseband frequency of 7 kHz and, in combination therewith, a higher order harmonic at 14 kHz due to doubling processes. Depending on the assembly's mechanical properties, non-harmonic frequencies may be excited in the rigid bodies along with harmonic frequencies.

In an embodiment, a frequency window bandwidth may be controllably associated with a center frequency. In one embodiment, for all center excitation frequencies, the same frequency window bandwidth may be selected (e.g., 200 Hz). In another embodiment, different frequency window bandwidths may be associated with two or more excitation center frequencies.

In an embodiment, the frequency window bandwidth that is concomitant with a range of central excitation frequencies may be selected such to be either overlapping or non-overlapping. Considering for instance a baseband frequency of 2 kHz and its harmonics comprising 4 kHz, 6 kHz, and 8 kHz, the frequency window bandwidth may be non-overlapping (i.e., < 2kHz), or overlapping (>2 kHz). In an embodiment, a frequency window bandwidth may be defined based on a multiplicative factor of the central excitation frequency. Hence, for a multiplicative factor >1, the frequency window bandwidths may be overlapping, whereas for a multiplicative factor < 1 the frequency window bandwidths may be non-overlapping. In an embodiment, CISA engine **150** may allow setting an upper and lower boundary for the frequency window bandwidth. In any case, the one or more baseband frequencies and, if present, the corresponding harmonics propagating in assembly **200** as a result of subjecting the assembly to one or more excitation frequencies, are analyzed by system **100** (e.g., by CISA engine **150**) for determining a state and/or a change in a state of a structural feature of assembly **200.**

For example, assembly **200** can be subjected to a center excitation frequency of 3000 Hz and, concomitantly, to an associated frequency window bandwidth of 400 Hz. In response, comparatively "strong" or "high-energy" frequency components may be measured at the excited frequency components 3050-3100 Hz and 6000 Hz. Since in the latter example the "strong" frequency components are in the range of up to 3100 Hz, a frequency window of +/- 100 Hz may be included for the analysis. In accordance with the above example, the acoustic energy at baseband frequency 3000+/- 100Hz may, for example, be compared against the acoustic energy obtained at the baseband's harmonics 6000 Hz +/- 100, and 9000 Hz +/- 100 for different assembly loading conditions.

In an embodiment, the near or reference sensor **106** may provide a feedback signal to CISA engine **150** according to which an input to the first transducer(s) **102** may be automatically adjusted. For example, the automatic adjustment may be used to standardize the excitation input produced by a transducer by taking into account noise factors such as varying or inter-individual soft-tissue presence in assembly **200,** bone geometry etc.

In some embodiments, the operating parameters of the one or more first and second transducers **102** and/or **104** may be controlled based on values measured by the one or more near and distant sensors **106** and **108**. The value(s) relating to the transducer operating parameters may be descriptive of and/or or relate to energy, phase, and/or frequency of mechanical waves to be excited in assembly **200**. Analogously, the value(s) relating to the signals produced by the sensors may, for example, be descriptive of and/or or relate to energy, phase, and/or frequency of mechanical waves propagating in assembly **200**. In some embodiments, the value(s) may be descriptive of an interference pattern generated in first rigid body **210** and/or second rigid body **220**.

In an embodiment, CISA engine **150** may be operative to determine a state and/or change of state of a structural feature of assembly **200,** such as of contact interface **230.** For example, CISA engine **150** may be operative to provide an input signal to a first transducer (e.g., transducer **102A**) for exciting the propagation of mechanical waves from implant **210** through contact interface **230** to bone structure **220.** CISA engine **150** may process and analyze the signals generated by reference sensor **106** and distant sensors **108** for determining a state and/or a change of state of a structural feature of assembly **200.** As already indicated herein, reference sensor **106** and distant sensor **108** are positioned to pick up physical stimuli of a mechanical wave propagating in bone structure **220** responsive to the excitation input provided at implant **210.** Additionally or alternatively, the input signal that is fed into the first transducer and the output signal produced by the distant sensor may be processed and analyzed by CISA engine **150** for determining a state and/or a change of state of a structural feature of assembly **200.** The electronic signal provided by distant sensor **108** provides an output signal. Optionally, the output signal produced by distant sensor **108** may be fed into the first transducer to provide a feedback.

For example, based on one or more non-conformity criteria, CISA engine **150** can provide an output indicative of the stability of assembly **200,** the state of the coupling and/or an output indicative of a reduction in conformity between the first and second surface of contact interface **230.** The one or more non-conformity criteria may be predetermined or determined in an adaptive manner. The non-conformity criterion may, for example, be set individually and/or adaptively for each patient. Optionally, the non-conformity criterion may be adapted while conducting measurements on the same patient.

In an embodiment, the non-conformity criterion may be set independent of or without requiring a generalized reference standard such as, for example, a medical "Gold Standard" and/or without requiring the tracking of individuals and/or groups over a period of time. Correspondingly, in an embodiment, system **100** may not have to be calibrated against a generalized reference standard. For example, with the use of system **100,** it may not be necessary to measure assembly parameters of a control group comprising dozens or even hundreds of patients at a point in time when the implants of the patients' assemblies could certainly be considered to be fixed to arrive at the generalized reference standard, and compare the reference standard against a measurement of a parameter relating to an assembly of a patient that is not a member of the control group. It may also not be necessary to repeatedly set up identical physiological loading conditions for examining the patient's prosthesis and/or articulating joint and logging the test measurements over a period of time, (e.g., every 7 days over the course of 12 weeks) to establish if there is a deviation, for example, from the reference standard or from a first measurement that was performed for the same individual at a point in time when the implant was known to stable.

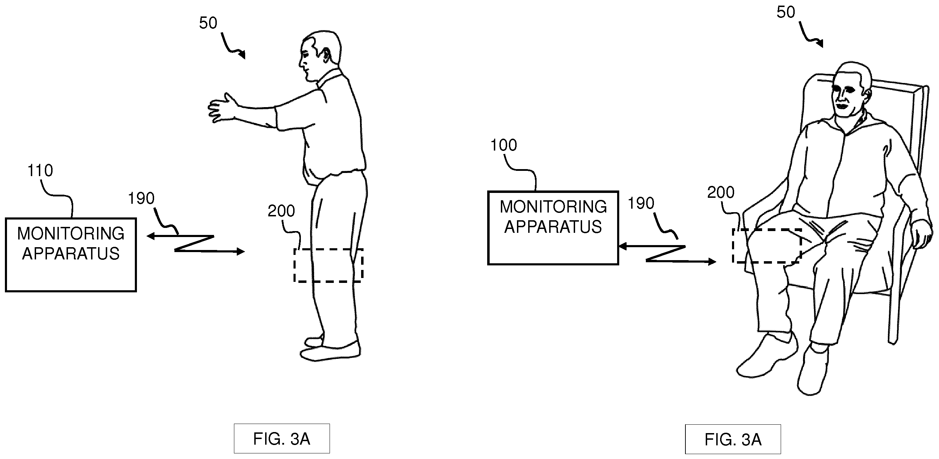

Additional reference is made to **FIG. 3A** and **3B.** The dashed box that is referenced by alphanumerical label **"200"** exemplarily and schematically indicates the location of assembly **200** in a patient **50** and, correspondingly, the region where the sensors and transducers of system **100** are located. In some embodiments, the sensors and transducers are mounted outside the patient's body, and in some other embodiments, they can be implanted in the patient's body.

Either way, with the use of system **100,** acoustic flux measurements conducted across an assembly of the same patient while the assembly is in relaxed and loaded condition, can be used for determining a state or change of state of a structural feature of the assembly in a single examination session without comparing the measurements against a reference standard, because when the assembly is considered to be "loaded", i.e., when the assembly is in the loaded condition, the assembly can be expected to always behave properly. For instance, a static joint such as the one shown in **FIG. 1A** will always behave as if it is fixed under loaded conditions (schematically exemplified in **FIG. 3A**), even if there is actually loosening of the static joint. Hence, by measuring acoustic flux across the contact interface of the assembly under different loading conditions ("loaded (loading) condition" shown in **FIG. 3A** and "relaxed (loading) condition" shown in **FIG. 3B**) and by comparing the measurements with each other, the state and/or a change of state of a structural feature of the assembly can be determined.

In an embodiment, CISA engine **150** may be operative to detect loosening of assembly **200** in a patient by subjecting assembly **200** to different loading conditions, and by comparing a value relating to a parameter of a mechanical wave that propagated through contact interface **230** under a first loading condition, against a value of a mechanical wave that propagated through contact interface **230** under a second loading condition that is different from the first loading condition. If the comparison yields a result that meets the non-conformity criterion, a diagnostic output may be provided indicating that assembly **200** is loosened. Such diagnostic output may be provided by CISA engine **150** via user interface **114.**

In one exemplary scenario where the assembly is comparatively stable, values relating to parameters of mechanical waves propagating through contact interface **230** while the assembly is loaded may be considerably different from values for a relaxed assembly. Conversely, if the assembly is comparatively unstable, e.g., due to a loosening of implant **210,** the values under the loaded and the relaxed condition may be comparatively equal. Considering for instance the above-noted example of a tibial implant, an relaxed condition may refer to a scenario in which the patient is sitting, as opposed to a loaded condition in which the patient is standing.

It should be noted that the above noted exemplary scenario and related assessments should by no means be construed limiting and may depend, for example, on the type of assembly. For instance, in another exemplary scenario, if an assembly is comparatively stable, values relating to parameters of mechanical waves propagating through contact interface **230** may be comparatively equal in both loaded and relaxed condition. On the other hand, if the same assembly is comparatively unstable, values obtained in the loaded condition may be significantly different from values obtained in the relaxed condition.

In an embodiment, a value of a parameter of a propagating mechanical wave that may be compared with respect to the non-conformity criterion that is based on an energy-ratio between the first harmonic and its base frequency. Optionally, such energy-ratio may form the basis for an implant-looseness metric that may be compared against the non-conformity criterion to yield a corresponding diagnostic output.

In an embodiments, the non-conformity criterion may be defined with respect to an interference pattern.

In an embodiment, the non-conformity criterion may relate to one or more difference and/or ratio threshold values of energy-ratios (or any other values relating to parameters of mechanical waves propagating from first rigid body **210** across contact interface **230** to second rigid body **220,** and/or vice versa) that were obtained under loaded and relaxed conditions. CISA engine **150** may for example determine, based on the ratio threshold values, a state and/or a change in state of contact interface **230** (such as stability and/or surface congruence; and/or a change in stability and/or surface congruence) and provide a corresponding diagnostic output. For example, if a comparison between energy-ratios yields a result that exceeds a certain threshold value, CISA engine **150** may provide a diagnostic output indicating that assembly **200** (and/or contact interface **230**) is/are stable. Conversely, if a comparison between energy-ratios yields a result which is equal or below the threshold value, CISA engine **150** may provide, correspondingly, a diagnostic output indicating that assembly **200** is unstable (e.g., that there is a certain incongruence between contacting surfaces forming contact interface **230.**

In an embodiment, the energy-ratio may be determined by computing the ratio of the average energy in the frequency bands around the baseband frequency $f_c$ and its first harmonic, e.g., as follows:

$$R^{f_c}_{1,0} = \frac{\sum_{f=2f_c-BW/2}^{2f_c+BW/2} A(f)^2}{\sum_{f'=f_c-BW/2}^{2f_c+BW/2} A(f')^2} \qquad (1)$$

where A(x) is the value of the spectral component at frequency x, and BW denotes the bandwidth. Exemplarily, the frequency window bandwidth may be $2/3f_c$. Considering, for example, a central excitation frequency of 3 kHz, the bandwidth of the resulting baseband frequencies is 2-4 kHz (having a corresponding central baseband frequency of 3 kHz) and the bandwidth of the first harmonic is 5-7 kHz (having a corresponding central baseband frequency of 6 kHz).

In an embodiment, a Fast-Fourier-Transform (FFT) is applied to transform a desired dataset in the time-domain into a desired frequency-domain dataset.

In an embodiment, the desired dataset in the time-domain may be obtained by collecting data descriptive of signal values of mechanical wave parameters over a relevant time period, e.g., during which most of the operably relevant signal values are generated. Alternatively, data descriptive of signal values may be collected over an extended time period. Data descriptive of signal values collected over the extended time period may include data descriptive of signal values which are at value "zero" (or irrelevant) for continuous time sections, as well as data descriptive of operably relevant signal values. Accordingly, the extended time period can also include the relevant time period. Data descriptive of signal values collected over the extended time period may be windowed, (e.g., using a Hanning window) to obtain the desired dataset in the time-domain for subsequent FFT transform. For example, a Hanning window of lengths 160, 100 and 90 points may be employed for 1500Hz, 3000Hz, and 4500Hz, respectively, followed by an FFT with 30k points. Windowing may be performed to cut away extended "zero" parts (i.e., tail signal components with comparatively little or no useful information) before and after a signal of interest, to reduce the computational power required for applying FFT. For instance, after cutting away or discarding the tailing "zero" parts, the FFT may be applied on, e.g., 3000 points instead of on, e.g., 100k points. Moreover, windowing may be performed to reduce or eliminate aliasing that could otherwise appear if now windowing was applied.

Considering the above-referenced energy-ratio metric, the threshold value may for example be defined as a 10-fold increase in the magnitude of the energy-ratios obtained under loaded and relaxed conditions. For example, if a change in the energy-ratios is less than 10-fold, CISA engine **150** may provide an output indicating that the corresponding assembly **200** is loosened. Conversely, if a change in the energy-ratios is 10-fold or higher, CISA engine **150** may provide an output indicating that assembly **200** is intact. The threshold values may range, for example, from 5-fold to 100-fold of energy-ratios.

In some embodiments, more than one threshold value may be defined for determining a state and/or a change of state of contact interface **230** which may be expressed, for example, by a level of correspondence, degree of conformity, non-conformity, stability, instability, congruence, incongruence, strength, stiffness and/or degree of looseness between at least two surfaces of contact interface **230,** e.g., on a scale ranging from 1 to 5. Numerical value "5" may for example indicate "highest stability" and numerical value "1" may indicate "very loose". Additional or alternative qualitative metrics may be employed.

In some embodiments, different sets of threshold values may be associated in system **100** to respective types of assemblies. In an embodiment, a user can indicate via user interface **114** the type of assembly that is about to be examined.

In some embodiments, different excitation and/or sensing routines may be implemented by system **100.** For instance, various automatic or semi-automatic mechanical wave excitation and/or sensing routines may be selected by the user and controlled by controller **160**. Optionally, controller **160** may automatically adjust the operating parameters of, e.g., a transducer, in accordance with a control input received from CISA engine **150.** CISA engine **150** may for example receive a reference signal produced by a reference sensor and determine, based on the received reference signal and, and further based on the signal produced by the distant sensor, a control (also: feedback) signal that is provided to controller **160.** Based on the feedback signal, controller **160** controls the operation of the ipsilateral transducer. In other words, the transducer's operating parameters may be controlled using a feedback loop.

In some embodiments, the at least one first transducer **102** and the at least one second transducer **104** may be activated in an alternating manner, each one for a certain period of time. Optionally, a set of first and second transducers **102** and **104** may be activated alternatingly, each set for a certain period time. Optionally, operating parameters of first transducer (s) **102** may differ from operating parameters of second transducer(s) **104.**

In some embodiments, first and second transducer(s) **102** and **104** may operate concurrently, at least during a certain time period. For instance, first transducer(s) **102** may be activated and operate during a first time period while second transducer(s) **104** are inactive. Past the first time period both first and second transducer(s) **102** and **104** may be operated for a second time period. Past the second time period, operation of first transducer(s) **102** may be stopped while second transducer(s) **104** may remain operational.

In some other embodiments, the operating parameters of first and/or second transducer(s) **102** and **104** may be automatically controlled, e.g., by controller **160** to change over time according to a control routine operating according to predefined operating parameters. Such control routine is herein referred to as "predefined control routine". The instructions for executing the control routine may be stored in memory **112**. For example, first and second transducer(s) **102** and/or **104** may be controlled so that an excitation frequency may either increase over certain time period from an initial frequency until an upper frequency limit is reached or decrease from an initial frequency over a certain time period until a lower frequency limit is reached. In another example, an excitation frequency may increase over a certain time period until an upper frequency limit is reached and then decrease over a certain time period until a lower frequency limit is reached, or vice versa. Analogously, the amplitude of the transducer excitation input may be increased or decreased. In an embodiment, the phase between two transducers may be controlled. For example, the phase between at least two of a plurality of first transducers **102** may be controlled, e.g., by controller **160.** Analogously, the phase between at least two of a plurality of second transducers **104** may be controlled. In some embodiments, the excitation phase between a first transducer **102** and a second transducer **104** may be controlled by CISA engine **150.** In view of the aforesaid, exemplarily, the phase between second transducers **104A** and **104B** may be controlled and/or the phase between second transducer **104A** and first transducer **102A** may be controlled.

In some embodiments, the predefined control routine may be used alongside the feedback loop. For instance, depending on the reference signal produced by the reference sensor, the predefined control routine may override the feedback loop, or the feedback loop may override the predefined control routine.

In some embodiments, the operating parameters of a control routine may be adjusted based on the received feedback signal. Such control routine may be referred to as an "adaptive" control routine.

In an embodiment, the at least one first transducer **102** and the at least one distant sensor **108** can be positioned to measure a flux of acoustic energy from the first to the second rigid body via the contact interface caused responsive to exciting the propagation of mechanical waves in the first rigid body. In an embodiment, the system is operative to measure a change in flux of acoustic energy. In an embodiment, a flux of acoustic energy or a change in flux (and/or any other value relating to mechanical wave parameters) may be determined based on values provided by distant sensor(s) **108,** near sensor(s) **106** and/or first transducer(s) **102.**

In some embodiments, system **100** may further comprise at least one third transducer **132** and/or at least one further sensor **134** that may be positioned in relatively close proximity to contact interface **230,** e.g., in comparison to the position of the other transducers and sensors, for example, to measure, at the contact interface, values of parameters relating to mechanical waves. In some embodiments, third transducer **132** and further sensor **134** may be co-located with contact interface **230.**

In some embodiments, a signal provided by a reference sensor may not only be used as a feedback to a transducer, but may additionally or alternatively be used for reducing or cancelling noise of a signal produced by another (e.g., distant) sensor. Considering for instance a second signal that is generated by distant sensor(s) **108** responsive to the sensing of mechanical wave propagation and which may have noise components introduced by the activation of the at least one first transducer **102,** near sensor(s) **106** may be employed to provide a reference signal for reducing or cancelling the noise components. For example, near sensor(s) **106** may be employed to sense noise components which may then be fully or partially removed (e.g., filtered or subtracted) from the second signal. Additional sources of noise that may be reduced or cancelled by such reference signal include, for example, soft tissue surrounding the sensor(s) and/or the transducer(s); movement of articulated assembly **200;** pressure applied on articulated assembly **200;** and/or environmental temperature fluctuations. In an embodiment, system **100** may comprise temperature sensors for temperature-calibrating the various components thereof. Additional or alternative sensors (not shown) may be employed including, for example, humidity sensors, light sensors, pressure sensors, , and/or the like. In some embodiments, pressure sensors (not shown) may be employed for measuring the load applied on articulated assembly **200.** Signals provided by such additional or alternative sensors may be employed for reducing or cancelling noise components.

According to some embodiments, at least one first transducer **102** and/or at least one second transducers **104** may comprise, for example, a piezoelectric transducer; a magneto-restrictive transducer; magneto-mechanical transducer; electromechanical transducers including, for example, a loudspeaker; an optical transducer including, for example, laser-based transducers; mechanical transducers including, for example, an impact hammer; and/or hydraulic-mechanical transducers.

At least one distant sensor **106** and/or at least one distal sensor **108** may comprise, for example, an accelerometer; a velocity sensor; a strain sensor; an acoustic sensor (e.g., an ultrasound sensor), and/or a displacement sensor; and may be based, for example, on a mechanics, optics (e.g., laser interferometry), hydraulics and/or electro-magnetism.

In some embodiments, the sensor(s) are implemented as contact and/or as noncontact sensor(s) for remote-sensing applications.

In some embodiments, transducers and/or the sensors are removably or non-removably attachable to a skin portion of a mammalian subject; a bone; an implant; bone pins; and/or any other tissue of, e.g., the mammalian subject. In some embodiments, the sensor(s) and/or transducer(s) may be biocompatible and/or fully or partially biodegradable and/or bioabsorbable. In some embodiments, a sensor and/or transducer can be part of or constitute a prosthesis and/or a bone. In other words, a prosthesis and/or bone may comprise or itself constitute a sensor and/or a transducer.

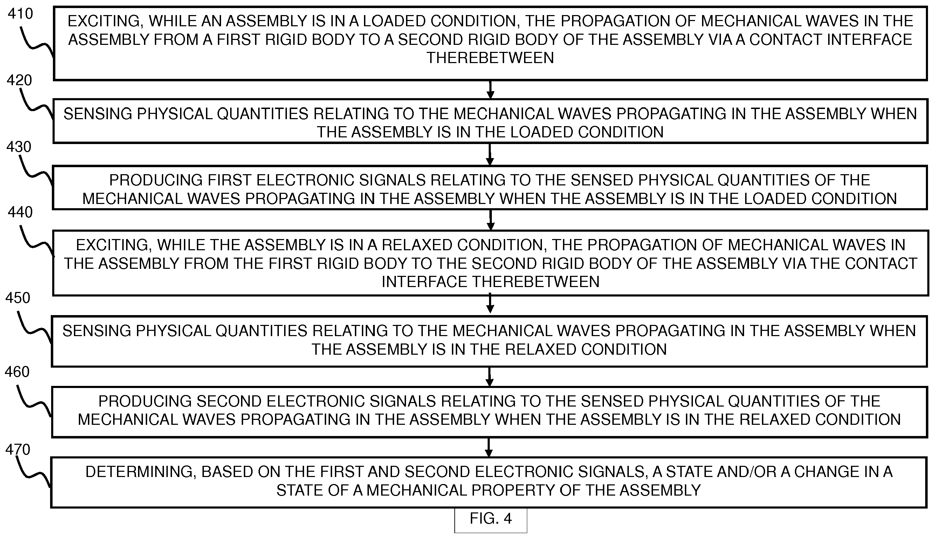

Additional reference is made to **FIG. 4.**

In embodiments, a method for monitoring one or more properties of an assembly of rigid bodies comprises, as indicated by step **410,** excite, while the assembly is in a loaded condition, the propagation of mechanical waves in the assembly from a first rigid body to a second rigid body of the assembly via a contact interface therebetween.

As indicated by step **420,** the method can further include sensing physical quantities relating to the mechanical waves propagating in the assembly when the assembly is in the loaded condition.

As indicated by step **430,** the method can include producing first electronic signals relating to the sensed physical quantities of the mechanical waves propagating in the assembly when the assembly is in the loaded condition.

As indicated by step **440,** the method can further include exciting, while the assembly is in a relaxed condition, the propagation of mechanical waves in the assembly from the first rigid body to the second rigid body of the assembly via the contact interface therebetween.

As indicated by step **450,** the method can include sensing physical quantities relating to the mechanical waves propagating in the assembly when the assembly is in the relaxed condition.

As indicated by step **460,** the method can further include producing second electronic signals relating to the sensed physical quantities of the mechanical waves propagating in the assembly when the assembly is in the relaxed condition.

As indicated by step **470,** the method can include determining, based on the first and second electronic signals, a state and/or a change in a state of a mechanical property of the assembly.

Optionally, a first electronic signal relating to the mechanical wave propagating in the first rigid body may comprise or constitute the electronic signal used for exciting the propagation of the mechanical waves. Optionally, a first electronic signal may be received from a near sensor that converts one or more physical stimuli relating to the mechanical waves propagating in the first rigid body into the first electronic signal. Optionally, a second electronic signal may be received from a distant sensor that converts one or more physical stimuli relating to the mechanical waves propagating in the second rigid body into the second electronic signal.

Prior to exciting the propagation of the mechanical waves, the method may in an embodiment comprise positioning the at least one first transducer and the at least one distant sensor such to enable the measuring of a flux of acoustic energy from the first to the second rigid body via the contact interface. Such flux is caused responsive to exciting, by the at least one first transducer, the propagation of mechanical waves in the first rigid body.

EXAMPLE:

Specimen preparation

This experiment used 12 sawbone tibiae (Model 3401, Sawbone Europe AB, Malmö, Sweden) as bone substitutes. After preliminary tests to confirm that these bones behaved indistinguishably, these tibiae were implanted with tibial plateaus of the type "balansys® RP" (Mathys AG, Bettlach, Switzerland) using original surgical equipment and methodology by an experienced operator.

To simulate the variety of clinically relevant implant behavior, four states of 3 bones each were prepared, of which one state mimics a stable implant while the other three states mimic various stages of implant loosening:

I) Cemented: The implantation was performed using standard procedure and bone cement. This state mimicked a cemented implant or a fully osseo-integrated, stable implant.

II) Press-fit: The implantation was performed without bone cement, following the standard procedure for a press-fit implant. This state mimicked a press-fit prosthesis directly after implantation and thus a slightly loose state as no osseo-integration has occurred yet.

III) Fibrous tissue: The implantation was performed by applying a small amount of silicone of type "Sikasil® C" (Sika Schweiz AG, Zurich, Switzerland) to the implant before performing the standard procedure for a press-fit implant. This state mimics a slightly loose prosthesis where failed osseo-integration has led to fibrous tissue between the implant and the bone. The silicone used was chosen because of its very similar properties to silicones used in other studies to simulate fibrous tissue (cf. to Okamura AM, Simone C, O'Leary MD. Force modeling for needle insertion into soft tissue. IEEE T Bio-Med Eng 51:1707-16,2004; and to Kerdok AE, Cotin SM, Ottensmeyer MP, Galea AM, Howe RD, Dawson SL. Truth cube: establishing physical standards for soft tissue simulation. Medical image analysis 7:283-91, 2003.)

Advanced loosening: The implantation was performed in the same way as for the press-fit state. The implant was then withdrawn and re-implanted five times. While no motion was visible by bare eye, this state mimicked a loosened prosthesis.

System:

The system consisted of three main parts: a piezo-electric actuator to create the wave, four accelerometers to measure the wave propagation, and a portable electronic unit to handle data acquisition and device control.

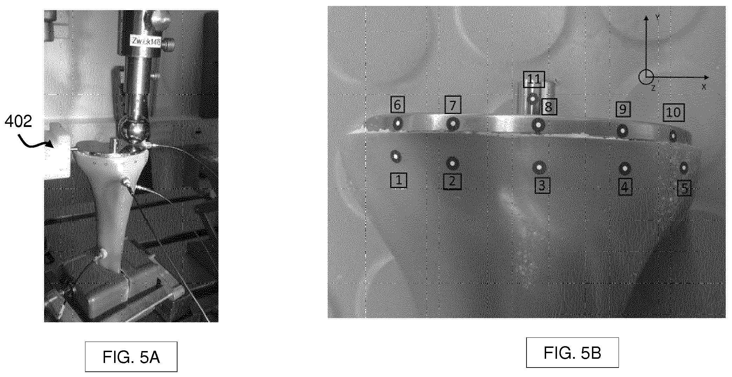

The actuator consisted of a P-841.20 piezo-stack (PI Ceramic GmbH, Lederhose, Germany) with a stainless steel attachment-head, both mounted in a custom made Teflon housing. The actuator was applied from the medial side to the implant, which is feasible procedure *in vivo* and can be seen in **FIG. 5A** and which is designated by reference numeral **402.** The strain gauges included in the piezo-stack are used to control the application pressure of the actuator during the experiments. The actuator was driven by a Gaussian enveloped sine wave with central frequency of 1.5/3.0/4.5 kHz and a Full-Width-Half-Maximum of typically 2/3 the central frequency after amplification through an E-617 high-power piezo-amplifier (PI GmbH & Co. KG, Karlsruhe, Germany).

The wave propagation was measured using four 4516 accelerometers (Brüel & Kjael GmbH, Pöcking, Germany). It is noted that reference numerals **1-11** depicted in **FIG. 5B** indicate the locations of markers for obtaining, based on optical measurements, additional information about a state of a contact interface such as looseness.. The signal generation for the actuator and the data acquisition from the accelerometers were handled by a USB-4431 data acquisition card (National Instruments, Austin, Texas) with a sampling rate of 96 kHz and an acquisition length of 200 samples, which corresponds to more than 6 times the cycle length for the central frequency of 3 kHz. The device was controlled by a laptop running in-house software, programmed in Labview 13 (National Instruments, Austin, Texas), which handled the signal generation and storage of raw measurement files for off-line analysis.

Loading Conditions:

The bones were mounted in an upright position using a custom-made mold and subjected to two different loading conditions, in which acoustic measurements were then taken:

Relaxed: The relaxed condition simulates a physiologically relaxed implant, e.g. present in sitting position.
Centrally loaded: A linear loading ramp of 0-2 kN was applied vertically to the tibial plateau at the central position and held at 2kN. This loading condition was applied to simulate the physiological loading conditions present during a one-legged stance. (Bergmann, G. (ed.), Charité Universitaetsmedizin Berlin (2008) "OrthoLoad" (retrieved Nov.

7, 2016 from http://www.orthoload.com).

Additionally, an acoustic measurement was performed in the relaxed configuration, in which the actuator was applied medially not to the implant but to the bone itself. As the excited waves, which were created in the material by the transducer, depend on the details of the transducer-material interaction, this procedure created a different kind of waves to be transmitted through the bone-implant interface compared to the excitation on the implant, thus offering additional information to differentiate implant states. This measurement condition is herein denoted as "bone" loading condition, to differentiate it from the normal "relaxed" loading condition with the actuator applied to the implant.

The loading device was a standard loading machine Zwick 1475 (Zwick Roell, Ulm, Germany) and the load was applied with a sphere-shaped stainless steel loading tip of radius 2.5 cm.

The optical system that was used for the visual tracking of the specimen motion under loading was an "Aramis 5M" digital image correlation system (GOM mbH, Braunschweig, Germany). 11 markers were placed onto the specimen at the positions respectively designated by reference numerals 1-11 (see **FIG. 5A**), so that their 3D-coordinates could be tracked by the visual tracking system during the loading cycles. Periodic calibration of the device was performed during the measurements to counteract noise influences e.g. changes in room temperature.

Acoustical measurements:

Analysis

The acoustic data were windowed using a Hanning window of lengths 160, 100, and 90 points, for the 1500Hz, 3000Hz, and 4500Hz measurements respectively. A fast-Fourier transform (FFT) with 30k points was used to transform the time data to frequency space, before the following quantities were computed:

Energy-ratio between the first harmonic and the base frequency: The ratio of the average energy in the frequency bands around the central frequency $f_c$ and its first harmonic were calculated using Equation 1.

Results:

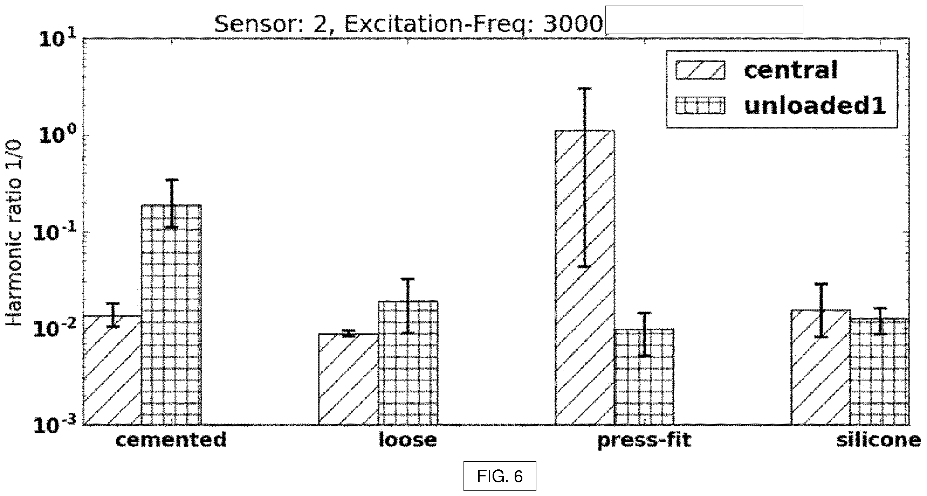

**FIGs. 5** and **6** present our findings for the energy ratio between the first harmonic and the base frequency. A strong influence of the loading condition can be seen in **Fig. 6** for the cemented and press-fit implant states, showing a 10-fold decrease and 100-fold increase respectively when comparing the unloaded measurement condition to the physiologically loaded one. Both loose and fibrous implant states show no clear change with respect to the loading condition. With the exception of sensor 2 at excitation frequency of 3 kHz, similar results can be seen for all the other sensors and excitation frequencies.

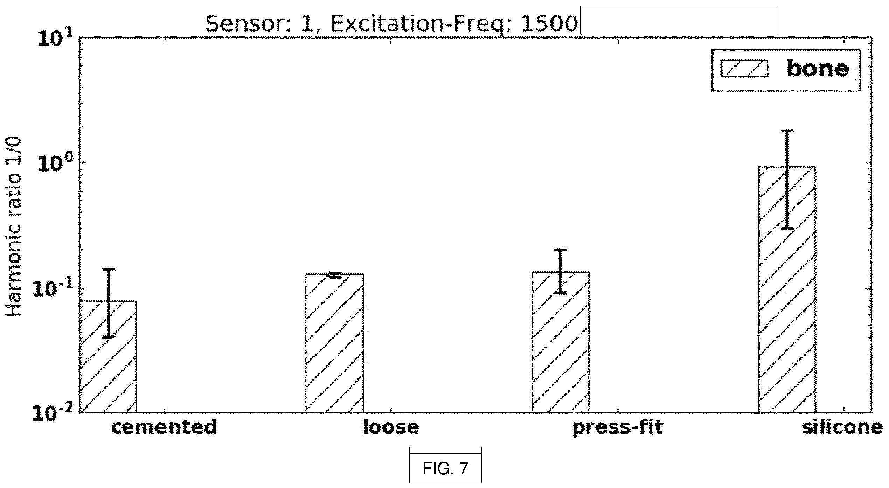

When the transducer **402** was positioned onto the bone instead of the implant in the unloaded configuration, the fibrous implant state showed a higher energy ratio than the other states, which can be seen in **FIG. 7.** This result appears for all four sensors, but only when the excitation frequency is 4.5 kHz.

The various features and steps discussed above, as well as other known equivalents for each such feature or step, can be mixed and matched by one of ordinary skill in this art to perform methods in accordance with principles described herein. Although the disclosure has been provided in the context of certain embodiments and examples, it will be understood by those skilled in the art that the disclosure extends beyond the specifically described embodiments to other alternative embodiments and/or uses and obvious modifications and equivalents thereof. Accordingly, the disclosure is not intended to be limited by the specific disclosures of embodiments herein.

Any digital computer can be configured or otherwise programmed to implement a method disclosed herein, and to the extent that a particular digital computer system is configured to implement such a method, it is within the scope and spirit of the disclosure. Once a digital computer system is programmed to perform particular functions pursuant to computer readable and executable instructions from program software that implements a method disclosed herein, it in effect becomes a special purpose computer particular to an embodiment of the method disclosed herein. The techniques necessary to achieve this are well known to those skilled in the art and thus are not further described herein. The methods and/or processes disclosed herein may be implemented as a computer program product such as, for example, a computer program tangibly embodied in an information carrier, for example, in a non-transitory tangible computer-readable or non-transitory tangible machine-readable storage device and/or embodied in a propagated signal, for execution by or to control the operation of a data processing apparatus including, for example, one or more programmable processors and/or one or more computers. The terms "non-transitory computer-readable storage device" and "non-transitory machine-readable storage device" encompasses distribution media, intermediate storage media, execution memory of a computer, and any other medium or device capable of storing for later reading by a computer program implementing embodiments of a method disclosed herein. A computer program product can be deployed to be executed on one computer or on multiple computers at one site or distributed across multiple sites and interconnected by one or more communication networks.

These computer readable and executable instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable and executable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable and executable instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

In the discussion, unless otherwise stated, adjectives such as "substantially" and "about" that modify a condition or relationship characteristic of a feature or features of an embodiment of the invention, are to be understood to mean that the condition or characteristic is defined to within tolerances that are acceptable for operation of the embodiment for an application for which it is intended.

It is important to note that the method is not limited to those diagrams or to the corresponding descriptions. For example, the method may include additional or even fewer processes or operations in comparison to what is described herein. In addition, embodiments of the method are not necessarily limited to the chronological order as illustrated and described herein.

Discussions herein utilizing terms such as, for example, "processing", "computing", "calculating", "determining", "establishing", "analyzing", "checking", "estimating", "deriving", "selecting", "inferring" or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other information storage medium that may store instructions to perform operations and/or processes. The term "determining" may also refer to "heuristically determining".

It should be noted that where an embodiment refers to a condition of "above a threshold", this should not be construed as excluding an embodiment referring to a condition of "equal or above a threshold". Analogously, where an embodiment refers to a condition "below a threshold", this should not to be construed as excluding an embodiment referring to a condition "equal or below a threshold". It is clear that should a condition be interpreted as being fulfilled if the value of a given parameter is above a threshold, then the same condition is considered as not being fulfilled if the value of the given parameter is equal or below the given threshold. Conversely, should a condition be interpreted as being fulfilled if the value of a given parameter is equal or above a threshold, then the same condition is considered as not being fulfilled if the value of the given parameter is below (and only below) the given threshold.

It should be understood that where the claims or specification refer to "a" or "an" element, such reference is not to be construed as there being only one of that element.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

Unless otherwise stated, the use of the expression "and/or" between the last two members of a list of options for selection indicates that a selection of one or more of the listed options is appropriate and may be made.

It is appreciated that certain features of the invention, which are, for clarity, described in the context of separate embodiments or example, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

It is noted that the term "exemplary" is used herein to refer to examples of embodiments and/or implementations, and is not meant to necessarily convey a more-desirable use-case.

As used herein, unless otherwise specified, the use of the ordinal adjectives "first", "second", etc., to describe like objects, merely indicate that different instances of like objects are being referred to, and are not intended to imply that the objects so described must be in a given sequence, temporally, in ranking, and/or in any other manner.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

While the invention has been described with respect to a limited number of embodiments, these should not be construed as limitations on the scope of the invention, but rather as exemplifications of some of the embodiments.

**Claims**

1. A system for monitoring one or more mechanical properties of an assembly of rigid bodies, comprising:

   at least one first transducer that is operative to excite mechanical waves that propagate from a first rigid body of the assembly via a contact interface to a second rigid body of the assembly;
   at least one distant sensor that is operative to sense one or more physical stimuli relating to the mechanical waves propagating in the second rigid body;
   a memory; and
   a processor that is operative to execute instructions stored in the memory to perform the following steps:

      receiving, while the assembly is under a loaded condition and also while the assembly is under a relaxed condition, electronic signals that relate to the mechanical waves propagating in the first and second rigid body; and
      determining, based on the received electronic signals, a state and/or a change in a state of a structural feature of the assembly

   wherein the electronic signals comprise: a) an electronic signal provided by the distant sensor;
   and at least one of the following: b) an electronic signal provided to the at least one first transducer to excite the propagation of mechanical waves; and c) an electronic signal provided by a near sensor located contralaterally to the distant sensor.

2. The system according to claim 1, wherein the structural feature is the contact interface.

3. The system according to any of the preceding claims, wherein the at least one first transducer and the at least one distant sensor are positioned on opposite sides of the contact interface.

4. The system according to any of the preceding claims, wherein the at least one first transducer and the at least one distant sensor can be positioned to measure a flux of acoustic energy from the first to the second rigid body via the contact interface, wherein the energy flux is generated by the first transducer exciting the propagation of mechanical waves in the first rigid body.

5. The system according to any of the preceding claims, wherein the system is operative to associate classifications with the received electronic signals as occurrences of one of the following: the loaded condition, and the relaxed condition;

   wherein the classification of the received electronic signals is performed automatically by the processor executing instructions stored in the memory, based on signals received from sensors that are coupled to a patient comprising the assembly; or
   based on an input provided by a medical professional via a user interface of the system.

6. The system according to any of the preceding claims, wherein the received electronic signals relate to energy, phase, and/or frequency of the propagating mechanical waves.

7. The system according to any of the preceding claims, further comprising at least one second transducer that is operative to excite mechanical waves that propagate from the second rigid body via the contact interface to the first rigid body.

8. The system of any of the preceding claims, wherein the at least one first and/or second transducers are operative to excite, in the assembly, the propagation of mechanical waves having a plurality of amplitudes, phases, and/or excitation frequencies.

9. The system according to any of the preceding claims, wherein the determined state and/or change of state of the contact interface includes a level of physical conformity between a surface of the first rigid body and a surface of the second rigid body forming the contact interface.

10. The system according to any of the preceding claims, wherein, based on a non-conformity criterion, the processor provides an output indicative of a state and/or change of state of the contact interface.

11. The system according to any of the preceding claims, wherein the at least one near and/or at least one distant sensor is employed to provide a reference value to reduce noise in the electronic signal of the at least one near and/or distant sensor and/or to control the at least one first and/or second transducer such to excite, in the respective rigid body, the propagation of a standardized mechanical wave.

12. A method for monitoring one or more mechanical properties of an assembly of rigid bodies, comprising:

exciting, while the assembly is in a loaded condition, the propagation of mechanical waves in the assembly from a first rigid body to a second rigid body of the assembly via a contact interface therebetween;
sensing physical quantities relating to the mechanical waves propagating in the assembly when the assembly is in the loaded condition;
producing first electronic signals relating to the sensed physical quantities of the mechanical waves propagating in the assembly when the assembly is in the loaded condition;
exciting, while the assembly is in a relaxed condition, the propagation of mechanical waves in the assembly from the first rigid body to the second rigid body of the assembly via the contact interface therebetween;
sensing physical quantities relating to the mechanical waves propagating in the assembly when the assembly is in the relaxed condition;
producing second electronic signals relating to the sensed physical quantities of the mechanical waves propagating in the assembly when the assembly is in the relaxed condition;
determining, based on the first and second electronic signals, a state and/or a change in a state of a mechanical property of the assembly,
wherein the first and second electronic signals each comprise: a) an electronic signal provided by the distant sensor; and at least one of the following: b) an electronic signal provided to the transducer to excite the propagation of mechanical waves; and c) an electronic signal provided by a near sensor located contralaterally to the distant sensor.

13. A computer program product comprising a program code for the execution of the method steps according to claim 12, wherein the computer program product is executed on a computer.

14. The use of a system according to any of the claims 1 to 11.

FIG. 1

EP 3 332 693 A1

FIG. 2

EP 3 332 693 A1

110

MONITORING
APPARATUS

190

50

200

FIG. 3A

100

MONITORING
APPARATUS

190

200

50

FIG. 3A

EP 3 332 693 A1

EP 3 332 693 A1

| 410 | EXCITING, WHILE AN ASSEMBLY IS IN A LOADED CONDITION, THE PROPAGATION OF MECHANICAL WAVES IN THE ASSEMBLY FROM A FIRST RIGID BODY TO A SECOND RIGID BODY OF THE ASSEMBLY VIA A CONTACT INTERFACE THEREBETWEEN |

| 420 | SENSING PHYSICAL QUANTITIES RELATING TO THE MECHANICAL WAVES PROPAGATING IN THE ASSEMBLY WHEN THE ASSEMBLY IS IN THE LOADED CONDITION |

| 430 | PRODUCING FIRST ELECTRONIC SIGNALS RELATING TO THE SENSED PHYSICAL QUANTITIES OF THE MECHANICAL WAVES PROPAGATING IN THE ASSEMBLY WHEN THE ASSEMBLY IS IN THE LOADED CONDITION |

| 440 | EXCITING, WHILE THE ASSEMBLY IS IN A RELAXED CONDITION, THE PROPAGATION OF MECHANICAL WAVES IN THE ASSEMBLY FROM THE FIRST RIGID BODY TO THE SECOND RIGID BODY OF THE ASSEMBLY VIA THE CONTACT INTERFACE THEREBETWEEN |

| 450 | SENSING PHYSICAL QUANTITIES RELATING TO THE MECHANICAL WAVES PROPAGATING IN THE ASSEMBLY WHEN THE ASSEMBLY IS IN THE RELAXED CONDITION |

| 460 | PRODUCING SECOND ELECTRONIC SIGNALS RELATING TO THE SENSED PHYSICAL QUANTITIES OF THE MECHANICAL WAVES PROPAGATING IN THE ASSEMBLY WHEN THE ASSEMBLY IS IN THE RELAXED CONDITION |

| 470 | DETERMINING, BASED ON THE FIRST AND SECOND ELECTRONIC SIGNALS, A STATE AND/OR A CHANGE IN A STATE OF A MECHANICAL PROPERTY OF THE ASSEMBLY |

FIG. 4

FIG. 5B

FIG. 5A

FIG. 6

FIG. 7

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 115 681 A (BOUHERAOUA AHMED [FR] ET AL) 26 May 1992 (1992-05-26) * column 2, last paragraph - column 3, paragraph 1; figure 2 * * column 5, lines 6-12 * * column 7, line 22 * ----- | 1-4,6, 8-14 | INV. A61B5/00 G01N29/07 A61F2/30 |
| X | US 5 115 808 A (POPOVIC GORAN [YU] ET AL) 26 May 1992 (1992-05-26) * figure 4 * ----- | 1-4,6, 8-14 | |
| X | US 2005/072236 A1 (HEYMAN JOSEPH S [US] ET AL) 7 April 2005 (2005-04-07) * figure 1 * ----- | 1-4,6, 8-14 | |
| X | US 5 172 591 A (BOHON W MARK [US]) 22 December 1992 (1992-12-22) * figure 3 * ----- | 1-4,6, 8-14 | |
| | | | TECHNICAL FIELDS SEARCHED (IPC) |
| | | | G01N A61B A61F |

The present search report has been drawn up for all claims

| Place of search | Date of completion of the search | Examiner |
|---|---|---|
| Berlin | 6 June 2017 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 16 20 2409

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 6, 9-14(completely); 8(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 16 20 2409

The Search Division considers that the present European patent application does not comply with the requirements of unity of invention and relates to several inventions or groups of inventions, namely:

1. claims: 1-4, 6, 9-14(completely); 8(partially)

    The electronic signal provided by a near sensor located contralaterally to the distant sensor
    ---

2. claim: 5

    The system is operative to associate classifications with the received electronic signals as occurrences of one of the following: the loaded condition, and the relaxed condition; wherein the classification of the received electronic signals is performed automatically by the processor executing instructions stored in the memory, based on signals received from sensors that are coupled to a patient comprising the assembly; or based on an input provided by a medical professional via a user interface of the system.
    ---

3. claims: 7(completely); 8(partially)

    The system comprises at least one second transducer that is operative to excite mechanical waves that propagate from the second rigid body via the contact interface to the first rigid body.
    ---

**EP 3 332 693 A1**

## ANNEX TO THE EUROPEAN SEARCH REPORT
## ON EUROPEAN PATENT APPLICATION NO.

EP 16 20 2409

06-06-2017

| Patent document cited in search report | | Publication date | Patent family member(s) | | Publication date |
|---|---|---|---|---|---|
| US 5115681 | A | 26-05-1992 | EP | 0395475 A1 | 31-10-1990 |
| | | | FR | 2646239 A1 | 26-10-1990 |
| | | | JP | H02297059 A | 07-12-1990 |
| | | | US | 5115681 A | 26-05-1992 |
| US 5115808 | A | 26-05-1992 | NONE | | |
| US 2005072236 | A1 | 07-04-2005 | CA | 2538133 A1 | 16-12-2004 |
| | | | EP | 1644696 A2 | 12-04-2006 |
| | | | US | 2005072236 A1 | 07-04-2005 |
| | | | WO | 2004109222 A2 | 16-12-2004 |
| US 5172591 | A | 22-12-1992 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **OKAMURA AM ; SIMONE C.** O'Leary MD. Force modeling for needle insertion into soft tissue. *IEEE T Bio-Med Eng,* 2004, vol. 51, 1707-16

- **KERDOK AE ; COTIN SM ; OTTENSMEYER MP.** Galea AM, Howe RD, Dawson SL. Truth cube: establishing physical standards for soft tissue simulation. *Medical image analysis,* 2003, vol. 7, 283-91
- Charité Universitaetsmedizin Berlin. 2008